# EUROPEAN PATENT APPLICATION

(11) **EP 4 454 713 A1**
(43) Date of publication of application: **30.10.2024**
(21) Application number: 23170745.6
(22) Date of filing: 28.04.2023
(51) Int. Cl.: A61Q 1/02, A61K 8/02, A61K 8/34, A61K 8/49

(54) **SEMI-PERMANENT TATTOO WITH IMPROVED APPLICATION**

(71) Applicant: inkbox ink Inc., Toronto, ON M5V 3G8 (CA)
(72) Inventor: MALLOV, Ian, Toronto (CA); KHALILI, Nazanin, Toronto (CA); LAM, Jolie, Toronto (CA)
(74) Representative: Peterreins Schley

(57) **Abstract**

In a first aspect the present disclosure relates to a temporary tattoo ink comprising one or more semi-permanent colorants, and one or more compounds selected from the group of 1,3-butylene glycol, pentylene glycol, hexylene glycol or mixtures thereof.

## Description

### Technical Field

The present invention relates to the field of tattoos. More specifically, the present invention relates to semi-permanent tattoos.

### Background

Temporary or semi-permanent tattoo or body inks have been used throughout human history to decorate the body. Generally, temporary or semi-permanent tattoos are transferred to the skin through the direct exposure of the skin to tattoo ink over a designated incubation period. The quality of the transferred image and its duration on the skin can depend on the ink distribution profile in the outer layer of the skin called the stratum corneum, or distribution profile in the epidermis. Current manufacturing processes for pre-fabricated tattoo designs include the utilization of topical formulations in many forms, including flexographic or gravure printing inks, stencils, and inkjet printers.

For example, semi-permanent tattoos may be applied using semi-permanent colorants, such as genipin and genipin derivatives. Genipin may chemically bind to the skin (mammalian, in particular human) resulting in a long-lasting blue coloration of the skin.

The semi-permanent colorant may be incorporated into an applicator, e.g. a sticker, to facilitate application of a semi-permanent tattoo by a user. The applicator may have a predefined pattern comprising the semi-permanent colorant, which defines the semi-permanent tattoo's design. For the manufacturing of an applicator for a semi-permanent tattoo, the semi-permanent colorants may be mixed with other compounds, such as solvents, to form a temporary tattoo ink. The temporary tattoo ink may be printed in the form of the predefined pattern onto an adhesive layer provided on a carrier liner. To apply the semi-permanent tattoo, the adhesive layer may then be placed on the part of the skin intended to display a semi-permanent tattoo, where the semi-permanent colorant may diffuse into the skin.

WO 2020/163830 A1 discloses such an applicator and suitable inks which include, besides Genipin as semi-permanent colorant, water, ethanol, diethylene glycol monoethyl ether (Transcutol^{®}) as liquid ink components. Inks based on these components are also commercialized by the present applicant.

However, it has been found that the resulting semi-permanent tattoos applied with such an applicator may have a patchy uneven appearance.

The present disclosure relates to improvements to semi-permanent tattoos.

### Summary

In a first aspect the present disclosure relates to a temporary tattoo ink comprising one or more semi-permanent colorants, and one or more compounds selected from the group of 1,3-butylene glycol, pentylene glycol, hexylene glycol or mixtures thereof.

In some embodiments according to the first aspect, the one or more compounds may comprise pentylene glycol.

In some embodiments according to the first aspect, the semi-permanent colorant may comprise genipin or a genipin derivative, in particular purified genipin.

In some embodiments according to the first aspect, the temporary tattoo ink may comprise one or more solvents, more specifically polar solvents, and in particular the solvents may be selected from the group of water, ethanol or mixtures thereof.

In some embodiments according to the first aspect, the temporary tattoo ink may comprise a surface active agent, more specifically the surface active agent may be a diol and in particular 1,2-hexanediol.

In some embodiments according to the first aspect, the temporary tattoo ink may comprise a matrix component, more specifically wherein the matrix component may be a polyol, even more specifically a disaccharide, and in particular trehalose.

In some embodiments according to the first aspect, the semi-permanent colorant may be present in an amount between about 1 wt.-% to about 20 wt.-%, more specifically between about 3 wt.-% to about 15 wt.-% and in particular between about 5 wt.-% to about 10 wt.-%, relative to the total weight of the temporary tattoo ink.

In some embodiments according to the first aspect, the one or more compounds may be present in an amount between about 1 wt.-% to about 30% wt.-%, more specifically between about 5 wt.-% to about 25 wt.-%, and in particular between about 10 wt.-% to about 20 wt.-%, relative to the total weight of the temporary tattoo ink.

In some embodiments according to the first aspect, the one or more solvents may be present in an amount between about 30 wt.-% to about 90% wt.-%, more specifically between about 50 wt.-% to about 85 wt.-%, and in particular between about 65 wt.-% to about 80 wt.-%, relative to the total weight of the temporary tattoo ink.

In some embodiments according to the first aspect, the solvent may comprise water and ethanol, more specifically the ratio between water and ethanol may be between about 1:1 to about 1:2, more specifically between about 1:1.3 to about 1:1.8.

In some embodiments according to the first aspect, the matrix component may be present in an amount between about 0.1 wt.-% to about 6 % wt.-%, more specifically between about 0.5 wt.-% to about 4 wt.-%, and in particular between about 1 wt.-% to about 3 wt.-%, relative to the total weight of the temporary tattoo ink.

In some embodiments according to the first aspect, the temporary tattoo ink may comprise less than 1 wt.-%, more specifically less than 0.1 wt.-% and in particular no or essentially no gelling agent, relative to the total weight of the temporary tattoo ink.

In a second aspect, the present disclosure relates to an applicator for applying a pattern comprising a semi-permanent colorant. The applicator comprises a carrier liner and an adhesive layer, and a pattern provided on and/or in the adhesive layer, wherein the pattern comprises a semi-permanent colorant and one or more compounds selected from the group of 1,3-butylene glycol, pentylene glycol, hexylene glycol or mixtures thereof.

In some embodiments according to the first aspect, the pattern may comprise a plurality of spots, wherein the plurality of spots may have an average maximum diameter between about 10 µm to about 500 µm, more specifically between about 50 µm to about 250 µm and in particular between about 75 µm to about 130 µm.

In some embodiments according to the second aspect, the applicator may comprise a release liner.

In some embodiments according to the second aspect, the carrier liner has a first face and a second face, wherein the adhesive layer may be provided on at least about 50%, more specifically at least 70% and in particular at least 90% of the second face, relative to the total surface of the second face.

In some embodiments according to the second aspect, the pattern may be provided on and/or in less than 95%, more specifically less than 90% and in particular less than 85% of the adhesive layer, relative to the total surface of the adhesive layer facing away from the carrier liner.

In some embodiments according to the second aspect, the adhesive layer may comprise, essentially consist or consist of a rubber adhesive.

In some embodiments according to the second aspect, the adhesive layer may comprise a pressure-sensitive adhesive.

In some embodiments according to the second aspect, the adhesive layer may be water-insoluble.

In some embodiments according to the second aspect, the carrier liner may comprise, essentially consist or consist of a water-resistant polymer or a waterproof polymer, in particular a waterproof polymer.

In some embodiments according to the second aspect, the carrier liner may be water-resistant or waterproof.

In some embodiments according to the second aspect, the carrier liner may have a thickness between about 10 µm to about 300 µm, more specifically between about 30 µm to about 150 µm and in particular between about 40 µm to about 100 µm.

In some embodiments according to the second aspect, the adhesive layer may have a thickness between about 5 µm to about 200 µm, more specifically between about 20 µm to about 100 µm and in particular between about 30 µm to about 80 µm.

In some embodiments according to the second aspect, the pattern comprising the semi-permanent colorant may comprise between about 0.01 wt.-% to about 30% wt.-%, more specifically between about 0.05 wt.-% to about 25 wt.-%, and in particular between about 0.1 wt.-% to about 20 wt.-%, of the one or more compounds, relative to the total weight of the pattern.

In some embodiments according to the second aspect, the pattern may comprise a matrix component, in particular wherein the matrix component comprises a polyol.

In some embodiments according to the second aspect, the polyol may have a molar mass of at least 100 g/mol, more specifically at least 200 g/mol, and in particular at least 300 g/mol. The matrix component may be solid at 0°C, more preferably a solid at 20°C, still more specifically a solid at 30°C, in particular solid at 45°C.

In some embodiments according to the second aspect, the pattern comprising the semi-permanent colorant may comprise between about 0.01 wt.-% to about 90% wt.-%, more specifically between about 0.05 wt.-% to about 85 wt.-%, and in particular between about 0.1 wt.-% to about 80 wt.-%, of the one or more solvents, relative to the total weight of the pattern.

In a third aspect, the present disclosure relates to an applicator obtained by applying a temporary tattoo ink on an adhesive layer provided on a carrier liner. The temporary tattoo ink comprises one or more semi-permanent colorants, and one or more compounds selected from the group of 1,3-butylene glycol, pentylene glycol, hexylene glycol or mixtures thereof.

In some embodiments according to the third aspect, the temporary tattoo ink may comprise one or more solvents, more specifically one or more polar solvents, and in particular the solvents may be selected from the group of water, ethanol or mixtures thereof.

In some embodiments according to the third aspect, applying the composition may be performed by printing, in particular inkjet printing.

In some embodiments according to the third aspect, obtaining the applicator may additionally comprise a heat treatment, in particular following the application of the temporary tattoo ink to the adhesive layer.

In some embodiments according to the third aspect, the heat treatment may be performed at a temperature between about 45°C to about 85°C, more specifically between about 55°C to about 75°C for a duration of between about 20 min to about 120 min, in particular between about 30 min to about 60 min.

In some embodiments according to the third aspect, obtaining the applicator may additionally comprise, in particular after the heat treatment, attaching a release liner to the adhesive layer, in particular by lamination.

In some embodiments according to the third aspect, the temporary tattoo ink may comprise between about 30 wt.-% to about 90 wt.-% of the one or more solvents, and/or between about 1 wt.-% to about 30 wt.-% of the one or more compounds, and/or between about 1 wt.-% to about 20 wt.-% of the one or more semi-permanent colorants.

In some embodiments according to the third aspect, the one or more compounds may comprise pentylene glycol.

In some embodiments according to the third aspect, the adhesive layer may comprise, essentially consist or consist of a rubber adhesive.

In a fourth aspect, the present disclosure relates to a method for manufacturing an applicator for applying a pattern comprising a semi-permanent colorant. The method comprises applying a temporary tattoo ink on an adhesive layer provided on a carrier liner, wherein the temporary tattoo ink comprises one or more semi-permanent colorants, and one or more compounds selected from the group of 1,3-butylene glycol, pentylene glycol, hexylene glycol or mixtures thereof.

In some embodiments according to the fourth aspect, the temporary tattoo ink may comprise one or more solvents, more specifically one or more polar solvents, and in particular the solvents may be selected from the group of water, ethanol or mixtures thereof.

In some embodiments according to the fourth aspect, applying the composition may be performed by printing, in particular inkjet printing.

In some embodiments according to the fourth aspect, obtaining the applicator may additionally comprise a heat treatment, in particular following the application of the temporary tattoo ink.

In some embodiments according to the fourth aspect, the heat treatment may be performed at a temperature between about 45°C to about 85°C, more specifically between about 55°C to about 75°C for a duration of between about 20 min to about 120 min, in particular between about 30 min to about 60 min.

In some embodiments according to the fourth aspect, obtaining the applicator may additionally comprise, in particular after the heat treatment, attaching a release liner to the adhesive layer, in particular by lamination.

In some embodiments according to the fourth aspect, the temporary tattoo ink may comprise between about 30 wt.-% to about 90 wt.-% of the one or more solvents, between about 1 wt.-% to about 30 wt.-% of the one or more compounds, and/or between about 1 wt.-% to about 20 wt.-% of the one or more semi-permanent colorants.

In some embodiments according to the fourth aspect, the one or more compounds may comprise pentylene glycol.

In some embodiments according to the fourth aspect, the adhesive layer may comprise, essentially consist or consist of a rubber adhesive.

In a fifth aspect the present disclosure relates to the use of one or more compounds selected from the group of 1,3-butylene glycol, pentylene glycol, hexylene glycol and mixtures thereof in a temporary tattoo ink for preventing or reducing transfer of the printed temporary tattoo ink onto a release liner.

### Brief Description of the Figures

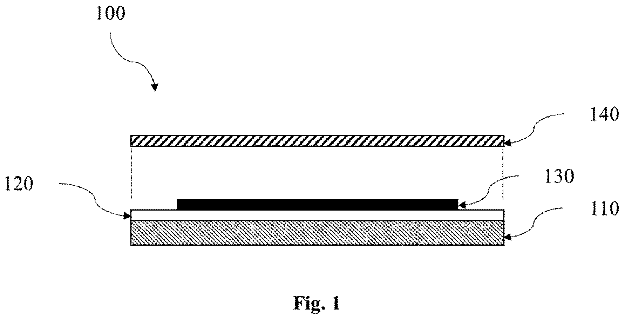

**Fig.1** shows an exploded- view drawing of an applicator according to the first aspect.

### Detailed Description

Hereinafter, a detailed description will be given of the present disclosure. The terms or words used in the description and the aspects of the present disclosure are not to be construed limitedly as only having common-language or dictionary meanings and should, unless specifically defined otherwise in the following description, be interpreted as having their ordinary technical meaning as established in the relevant technical field. The detailed description will refer to specific embodiments to better illustrate the present disclosure, however, it should be understood that the presented disclosure is not limited to these specific embodiments.

Semi-permanent colorants may be incorporated into applicators, e.g. stickers, to facilitate application of a semi-permanent tattoo by a user. The applicator may have a predefined pattern comprising the semi-permanent colorant, which defines the semi-permanent tattoo's design. For the manufacturing of an applicator for a semi-permanent tattoo, the semi-permanent colorants may be mixed with other compounds, such as solvents, to form a temporary tattoo ink. The temporary tattoo ink may be printed in the form of the predefined pattern onto an adhesive layer provided on a carrier liner. To apply the semi-permanent tattoo, the adhesive layer may then be placed on the part of the skin intended to display a semi-permanent tattoo, where the semi-permanent colorant may diffuse into the skin.

The application of semi-permanent tattoos using the aforementioned applicators offers multiple advantages. Firstly, it allows the user to apply a semi-permanent tattoo in a single step, without considerable effort by the user. Additionally, applying the temporary tattoo ink to the adhesive layer by printing allows for high-throughput manufacturing with precise designs. In addition, the aforementioned tattoos may not require wetting by water for the transfer of the pattern design to the skin.

However, it has been found that the semi-permanent colorant may not diffuse from the applicator into the skin evenly. In fact, the resulting semi-permanent tattoo may appear patchy. The term "patchy semi-permanent tattoo" herein refers to a semi-permanent tattoo comprising regions of (unintended) color intensity differences, in particular with regards to the obtained result on the skin. Without wishing to be bound by theory, it is believed that this may occur due to an interaction of the adhesive layer, the temporary tattoo ink and a release liner.

It has been surprisingly found that by including one or more compounds selected from the group of 1,3-butylene glycol, pentylene glycol, hexylene glycol or mixtures thereof into the temporary tattoo ink applied to the adhesive layer, the patchiness of the resulting tattoo may be significantly reduced.

Without wishing to be bound by theory, it is assumed that the aforementioned alkyl glycols have slight surfactant properties that may be beneficial in facilitating a homogeneous spread of the (dried) ink on the adhesive layer. This belief is based on the following observation: The afore-mentioned tattoo patch comprises a carrier liner, an adhesive layer on which the tattoo pattern is printed and a release liner. A release liner is a material layer, e.g. a sheet of paper, attached to the adhesive layer opposite of the carrier liner. The release liner may protect the adhesive layer and the pattern disposed thereon during storage and transport. The release liners are configured to be easily releasable, however, they need to adhere sufficiently to prevent premature release. It has been found that after removal of the release liner, the quality of the pattern may be degraded, in particular the pattern may appear patchy when backlighted with light in the visible spectrum. In particular, some areas of the pattern may be transparent, whereas other parts of the pattern may appear opaque when backlighted. It has been found that the observable patchiness of the pattern is directly correlated to a patchiness of the resulting semi-permanent tattoo. The color of the semi-permanent colorant may develop over time and as a result, the patchiness of the tattoo may only be visible after 12 hours to 24 hours. In particular, areas of the pattern that appeared opaque when backlighted, resulted in an area of the semi-permanent tattoo exhibiting a lower color intensity, compared to those areas where a transparent pattern was applied to. In some cases, after removal of the release liner (almost) all of the pattern appeared opaque, which led to a semi-permanent tattoo of lower color intensity, compared to those applied by a transparent pattern. Accordingly, it is believed that the aforementioned glycols already provide their benefit on the ink when applied to the adhesive layer and do not primarily act on the skin in a cosmetic mode of action (skin penetration, moisturizing effect, etc.).

Furthermore, the aforementioned glycols may allow providing a temporary tattoo ink that is still suitable for printing, in particular inkjet printing, and for skin contact.

### Temporary tattoo ink

Accordingly, in a first aspect the present disclosure relates to a temporary tattoo ink comprising one or more semi-permanent colorants, and one or more compounds selected from the group of 1,3-butylene glycol, pentylene glycol, hexylene glycol or mixtures thereof.

In some embodiments according to the first aspect, the one or more compounds may comprise in particular pentylene glycol. In some embodiments according to the first aspect, the one or more compounds may be present in an amount between about 1 wt.-% to about 30% wt.-%, more specifically between about 5 wt.-% to about 25 wt.-%, and in particular between about 10 wt.-% to about 20 wt.-%, relative to the total weight of the temporary tattoo ink.

The term "colorant" is well-known and i.a. attributed its common meaning in the art. Additionally or alternatively, the term "colorant" may refer to a substance that changes the color of reflected or transmitted light as the result of wavelength-selective absorption. As used herein, "color" refers to wavelengths of electromagnetic radiation visible to the human eye. The term "semi-permanent colorant" is well-known and i.a. attributed its common meaning in the art. Additionally or alternatively, the term "semi-permanent colorant" may refer to a colorant that penetrates one or more layers of the skin and is unable to be removed from the skin without physical disruption or natural desquamation of the skin. For example, a semi-permanent colorant may penetrate the stratum corneum and/or epidermis and react with other molecules, e.g., other colorant molecules and/or other molecules present in the stratum corneum and/or epidermis, such that the semi-permanent colorant is immobilized within the stratum corneum and/or epidermis. For example, the semi-permanent colorant may cross-link with collagen found in the stratum corneum and/or epidermis. In some embodiments according to the first aspect, the semi-permanent colorant may react with other molecules, e.g., other colorant molecules and/or other molecules present in the stratum corneum and/or epidermis, to form a molecule that is too large to diffuse out of the stratum corneum and/or epidermis. In some embodiments according to the first aspect, wherein the semi-permanent colorant penetrates the stratum corneum and/or epidermis, the colorant residence time is determined by the natural skin desquamation process. In some embodiments according to the first aspect, the semi-permanent colorant cannot be washed off, for example, by water, soap, and/or isopropanol. In some embodiments according to the first aspect, a semi-permanent colorant includes a natural or synthetic pigment or dye. In some embodiments according to the first aspect, a semi-permanent colorant can include a colorant precursor, for example, a molecule, such as genipin, that expresses color upon reaction with one or more other molecules.

In some embodiments according to the first aspect, the one or more semi-permanent colorant may comprise a colorant precursor. In some embodiments according to the first aspect, the colorant precursor is genipin. Accordingly, in some embodiments according to the first aspect, the semi-permanent colorant may comprise genipin or a genipin derivative, in particular purified genipin. As used herein, "genipin" refers to genipin extract, partially purified genipin extract, and/or purified genipin, as described further herein. The term "purified genipin" may relate to a semi-permanent colorant comprising at least about 90 wt.-%, more specifically at least about 95 wt.-% and in particular at least about 98.5 wt.-% genipin, relative to the total weight of the semi-permanent colorant. Genipin is derived from an iridoid glycoside called geniposide, which is present in nearly 40 plant species. Genipin may be derived for example from the plant Genipa americana, in particular its fruits. The plant genipa americana is also known as jagua, caruto, huito, jenipapo, genipapa, guaitil, tapaculo, yigualti, shagua, xagua, maluco, vito, ñandipá or bi. Genipin may also be derived for example from Gardenia Jasminoides, in particular its fruits. Genipa is also sold under the names Jagua-extract or Genipa-food extract. Additionally, genipin is a precursor to gardenia blue that undergoes a free radical polymerization and/or oligomerization reaction upon exposure to amine groups to form a blue colorant (see, for example, Touyama et al., Chem. Pharm. Bull. 42:668-673, 1994; and Touyama et al., Chem. Pharm. Bull. 42(8): 1571-1578, 1994; both of which are incorporated by reference herein in their entireties). When placed in contact with the skin, genipin can react with the amines in the skin to generate the polymer-based and/or oligomer-based blue color in situ. The penetration profile of genipin can dictate the color concentration profile in the stratum corneum and/or epidermis as the polymer-based and/or oligomer-based blue colorant is too large to penetrate the skin at relevant concentrations.

In some embodiments according to the first aspect, the semi-permanent colorant may be present in an amount between about 1 wt.-% to about 20 wt.-%, more specifically between about 3 wt.-% to about 15 wt.-% and in particular between about 5 wt.-% to about 10 wt.-%, relative to the total weight of the temporary tattoo ink.

In some embodiments according to the first aspect, the temporary tattoo ink may comprise one or more solvents, more specifically one or more polar solvents, and in particular the solvents may be selected from the group of water, ethanol or mixtures thereof. The solvents may dissolve the semi-permanent colorant, e.g. genipin and/or other components, such as matrix components. The solvents, in particular their amounts, may also be adjusted to adjust the viscosity of the temporary tattoo ink . In some embodiments according to the first aspect, the one or more solvents may be present in an amount between about 30 wt.-% to about 90% wt.-%, more specifically between about 50 wt.-% to about 85 wt.-%, and in particular between about 65 wt.-% to about 80 wt.-%, relative to the total weight of the temporary tattoo ink. In some embodiments according to the first aspect, the solvent may comprise water and ethanol, more specifically the ratio between water and ethanol may be between about 1:1 to about 1:2, more specifically between about 1:1.3 to about 1:1.8. In some embodiments according to the first aspect, the temporary tattoo ink may comprise less than 1 wt.-%, more specifically less than 0.1 wt.-% and in particular no or essentially no transcutol, relative to the total weight of the temporary tattoo ink.

The temporary tattoo ink may comprise one or more additional colorants, in particular one or more temporary colorants. The term "temporary colorant" is well-known and i.a. attributed its common meaning in the art. Additionally or alternatively, the term "temporary colorant" may refer to a colorant that sits on top of the skin or, if it penetrates the skin, can diffuse out of the skin or can be washed off by, for example, water, soap, and/or isopropanol. As mentioned above, semi-permanent colorants such as genipin, may require time to (fully) develop their color. The additional colorant may visualize the applied temporary tattoo directly after application. In some embodiments according to the first aspect, the additional colorant, in particular the temporary colorant, may be present in an amount between about 0.05 wt.-% to about 1 wt.-%, more specifically between about 0.1 wt.-% to about 0.5 wt.-%, relative to the total weight of the temporary tattoo ink. In some embodiments according to the first aspect, the one or more temporary colorants may be selected from the group consisting of: iron oxide black (Fe₃O4), iron oxide (FeO), carbon, logwood, ochre, cinnabar (HgS), cadmium red (CdSe), iron (III) oxide (Fe₂O₃), naphthol-AS pigment, disazodiarylide, disazopyrazolone, cadmium seleno-sulfide, cadmium yellow (CdS, CdZnS), curcuma yellow, chrome yellow (PbCrO₄), disazodiarylide, chromium oxide (Cr₂O₃), malachite [Cu₂(CO₃)(OH)₂], a ferrocyanide, a ferricyanide, lead chromate, monoazo pigment, Cu/Al phthalocyanine, Cu phthalocyanine, azure blue, cobalt blue, Cu-phthalocyanine, manganese violet (manganese ammonium pyrophosphate), an aluminum salt, quinacridone, dioxazine/carbazole, lead white (lead carbonate), titanium dioxide (TiO₂), barium sulfate (BaSO₄), zinc oxide, anthraquinone dyes and derivatives, annatto, caramel, β-carotene, bismuth citrate, disodium EDTA copper, potassium sodium copper chlorophyllin, dihydroxyacetone, bismuth oxychloride, guaiazulene, henna, ferric ammonium ferrocyanide, ferric ferrocyanide, chromium hydroxide green, chromium oxide greens, guanine, lead acetate, pyrophillite, mica, silver, titanium dioxide, aluminum powder, bronze powder, copper powder, ultramarines, manganese violet, zinc oxide, luminescent zinc sulfide, D&C Black Nos. 2 and 3, FD&C Blue No. 1 (e.g., acid blue 9), D&C Blue No. 4, D&C Brown No. 1, FD&C Green No. 3, D&C Green Nos. 5, 6, and 8, D&C Orange Nos. 4, 5, 10 and 11, FD&C Red Nos. 4, D&C Red Nos. 6, 7, 17, 21, 22, 27, 28, 30, 32, 33, 34, 36 and 40, D&C Violet No. 2, Ext. D&C Violet No.2, FD&C Yellow Nos. 5 and 6, D&C Yellow Nos. 7, 8, 10 and 11, and Ext. D&C Yellow No. 7. In some embodiments according to the first aspect, the temporary colorant does not contain an amine group. In some embodiments according to the first aspect, the temporary colorant is acid blue 9.

The temporary tattoo ink according to the first aspect may be used to print a pattern on an applicator. Accordingly, in some embodiments according to the first aspect, the temporary tattoo ink may be suitable for printing, in particular inkjet printing. In some embodiments according to the first aspect, the temporary tattoo ink may be an inkjet printer ink composition. The temporary tattoo ink may comprise additional ingredients which may improve its printability, e.g. by adjusting its viscosity and/or surface tension. In some embodiments according to the first aspect, the composition may be configured to have (or may have) a viscosity from about 1 to about 7 centipoise at 20°C. In some embodiments according to the first aspect, the composition may be configured to have (or may have) a viscosity from about 3 to about 6 centipoise at 20°C. In some embodiments according to the first aspect, the composition may be configured to have (or may have) a surface tension from about 20 to about 35 dyne/cm, more specifically between about 25 to 30 dyne/cm. In some embodiments according to the first aspect, the temporary tattoo ink may comprise less than 1 wt.-%, more specifically less than 0.1 wt.-% and in particular no or essentially no gelling agent, relative to the total weight of the temporary tattoo ink. In some embodiments according to the first aspect, the temporary tattoo ink may comprise less than 1 wt.-%, more specifically less than 0.1 wt.-% and in particular no or essentially no adhesive, relative to the total weight of the temporary tattoo ink. Gelling agents and adhesives may significantly increase the viscosity of the temporary tattoo ink making it unsuitable for printing, in particular inkj et printing. The viscosity may be measured for example using a Brookfield Ametek DVE Viscometer, model no. DVEELVTJ0 with a UL adaptor for viscosities lower than 10 centipoise. The surface tension and contact angle may be measured using a Kruss Force Tensiometer using the du Noüy ring method according to ASTM D1331-20.

In some embodiments according to the first aspect, the temporary tattoo ink may comprise a surface active agent. The term "surface-active agent" is well known and i.a. attributed its common meaning in the art. Additionally or alternatively, the term "surface-active agent" may relate to a substance that tends to reduce the surface tension of a liquid in which it is dissolved. Non-limiting examples of a surface-active agent include an alkylbenzene sulfonate, an alkyl sulfate, an alkyl ether sulfate, a soap, an ethoxylate, an alkyl alcohol, a lignosulfonate, and a triglyceride. For example, a surface-active agent can include sodium dodecyl benzenesulfonate, lauryl sulfate, di-alkyl sulfosuccinate, dimethyl ether of tetradecyl phosphonic, lauryl monoethanol, glycerol diester (diglyceride), dodecyl betaine, abietic acid, polyethoxylated octyl phenol, 1,2-hexanediol (e.g., OPTIPHEN^{®} HD), and sorbitan monoester. In some embodiments according to the first aspect, the surface-active agent may be a diol and in particular 1,2-hexanediol. The surface-active agent, in particular the 1,2-hexanediol may reduce the surface tension of the temporary tattoo ink, which may improve the printing quality of the ink. Further, the surface-active agent, in particular the 1,2-hexanediol, may improve the skin penetration of the semi-permanent colorant. In some embodiments according to the first aspect, the surface active agent may be present in an amount between about 0.1 wt.-% to about 6 % wt.-%, more specifically between about 0.5 wt.-% to about 4 wt.-%, and in particular between about 1 wt.-% to about 3 wt.-%, relative to the total weight of the temporary tattoo ink.

In some embodiments according to the first aspect, the temporary tattoo ink may comprise a matrix component. In some embodiments according to the first aspect, the matrix component may comprise a polyol. The matrix component may provide mechanical stability to the pattern. Further, the semi-permanent colorant may be partially embedded in the matrix component in the final applicator, which may reduce the rate of semi-permanent colorant diffusion onto the release liner. The matrix component may release the semi-permanent colorant when applied to skin, e.g. by being slightly moistened by sweat. Further, the matrix component may increase the resolution of the printed image. Without wishing to be bound by theory, the matrix component may form hydrogen bonds with the semi-permanent colorant in the ink used for printing and/or during the drying process, which may reduce the mobility of the semi-permanent colorant and thereby blurring of the printed image, e.g. by preventing the semi-permanent colorant to diffuse on or into the adhesive layer, in particular diffuse along the surface of the adhesive layer.

A polyol as used herein means an alcohol comprising at least three hydroxyl groups, in particular at least four hydroxyl groups. A polyol may include, for example, a mono-saccharide, di- saccharide, an oligo-saccharide such as a tri-saccharide, sugar alcohol or other short chain polymeric polyol. Optionally, a polyol as used herein may include polyol derivatives, which may have other hydrophilic groups incorporated therein, such as ethylene oxide derivatives of hydroxyl groups.

In some embodiments according to the first aspect, the polyol (of the matrix component) may be a polyol capable of redispersing in aqueous media, in particular the polyol may be water-soluble. The term "water-soluble polyol" within this disclosure may refer to a polyol with a water-solubility of more than 0.5 g/dL, more specifically more than 10 g/dL and in particular more than 50 g/dL, at 20°C. As mentioned above, the matrix component may release the semi-permanent colorant when moistened by sweat. Without wishing to be bound by theory, the matrix component when comprising a polyol may be partially solubilized when absorbing water, e.g. from sweat, allowing a higher rate of diffusion of the semi-permanent colorant towards the skin. Still, without wishing to be bound by theory, the matrix component may bind the semi-permanent colorant by forming hydrogen bonds with it, which are solubilized by water permeating into the pattern, in turn allowing for an increased movability of the semi-permanent colorant. The matrix component may also improve the transfer of the pattern to the skin, by acting as hygroscopic substance, in turn drawing sweat from the skin.

In some embodiments according to the first aspect, the polyol may have a molar mass of at least 100 g/mol, more specifically at least 200 g/mol, and in particular at least 300 g/mol. The matrix component may be solid at 0°C, more preferably a solid at 20°C, still more specifically a solid at 30°C, in particular solid at 45°C. In some embodiments according to the first aspect, the polyol may be solid at 0°C, more preferably a solid at 20°C, still more specifically a solid at 30°C, in particular solid at 45°C.

In some embodiments according to the first aspect, the matrix component may be selected from the group consisting of: a sugar, a sugar alcohol, and a polymer. In some embodiments according to the first aspect, the sugar may be selected from the group consisting of: trehalose, glucose, fructose, galactose, ribose, and xylose, trehalose, sucrose, lactotrehalose, galactotrehalose, 6-azidotrehalose, maltose, lactose, lactulose, cellobiose, chitobiose, a starch, glycogen, a cellulose, and a chitin. In some embodiments according to the first aspect, the matrix component may be trehalose. In some embodiments according to the first aspect, the matrix component may be present in an amount between about 0.1 wt.-% to about 6 % wt.-%, more specifically between about 0.5 wt.-% to about 4 wt.-%, and in particular between about 1 wt.-% to about 3 wt.-%, relative to the total weight of the temporary tattoo ink. The one or more compounds mentioned above may also aid in solubilizing the semi-permanent colorant. When the solubilization of the semi-permanent colorant is improved, its distribution and embedding in the matrix component may be improved, which may also result in less patchiness, in particular after longer storage durations.

In some embodiments according to the first aspect, the temporary tattoo ink may further comprise one or more preservatives. In some embodiments according to the first aspect, the one or more preservatives may be present in an amount of about 0.5 wt.-% to about 1.5 wt.-%, relative to the total weight of the temporary tattoo ink. In some embodiments according to the first aspect, the one or more preservatives may be present in an amount of about 1 wt.-%, relative to the total weight of the temporary tattoo ink. In some embodiments according to the first aspect, the one or more preservatives may be selected form the group consisting of: ascorbic acid, an ascorbate, a palmitate, citric acid, a benzoate, a benzoic acid, a propionate, propionic acid, a sorbate, sorbic acid, a salicylic acid, a salicylate, hexa-2,4-dienoic acid, a hexa-2,4-dienoate, formaldehyde, a formaldehyde releaser, formic acid and its salts, 3-acetyl-6-methylpyran-2,4-(3H)-dione and its salts, 3,3'-dibromo-4,4'-hexamethylenedioxydibenzamidine and its salts, thiomersal, phenylmercuric salts, undec-10-enoic acid and its salts, 1,3-bis (2-ethylhexyl) hexahydro-5-methyl-5-pyrimidine, 5-bromo-5-nitro-1,3-dioxane, bronopol, 2,4-dichlorobenzyl alcohol, 1-(4-chlorophenyl)-3-(3,4-dichlorophenyl) urea, chlorocresol, chloroxylenol, 5-chloro-2-(2,4- dichlorophenoxy) phenol, N,N"-methylenebis[N'-[3 -(hydroxymethyl)-2,5- dioxoimidazolidin-4-yl]urea], polyaminopropyl biguanide, methenamine, quaternium-15, climbazole, DMDM hydantoin, benzyl alcohol, 1-Hydroxy-4-methyl-6-(2,4,4-trimethylpentyl)-2-pyridon, piroctone olamine, bromochlorophene, o-cymen-5-ol, chlorophene, chloroacetaminde, methylchloroisothiazolinone, methylisothiazolinone, phenoxyisopropanol, chlorhexidine, chlorhexidine diacetate, chlorhexidine digluconate, chlorhexidine dihydrochloride, dimethyl oxazolidine, behentrimonium chloride, cetri-monium bromide, cetrimonium chloride, laurtrimonium bromide, laurtrimonium chloride, steartrimonium bromide, steartrimonium chloride, diazolidinyl urea, hexamidine, hexamidine diisethionate, hexamidine paraben, glutaral, 7-ethylbicyclooxazolidine, chlorphenesin, sodium hydroxymethylglycinate, silver chloride, benzethonium chloride, benzalkonium chloride, benza-lkonium bromide, benzalkonium saccharinate, benzylhemiformal, iodopropynyl butylcarbamate, biphenyl-2-ol and its salts, pyrithionine zinc, an erythorbate, a nitrite, ethylenediaminetetraacetic acid (EDTA), butylated hydroxyanisole (BHA), butylated hydroxytoluene (BHT), capryllic acid, dilauryl thiodipropionate, erythorbic acid, gum guaiac, methylparaben, a sulfite, a bisulfite, a metabisulfite, propyl gallatepy, propylparaben, stannous chloride, sulfur dioxide, thiodipropionic acid, an isothiazoline, a paraben, phenoxyethanol, ethylhexylglycerin, a glycols, and a tocopherol. In some embodiments according to the first aspect, the one or more preservatives may be phenoxyethanol and ethylhexylglycerin. A 90:10 mixture of phenoxyethanol and ethylhexylglycerin is available under the brand name EUXYL^{®} PE9010, by the company Ashland Global Speciality Chemical Inc., USA.

### Applicator

In a second aspect, the present disclosure relates to an applicator for applying a pattern comprising a semi-permanent colorant. The applicator comprises a carrier liner and an adhesive layer, and a pattern provided on and/or in the adhesive layer, wherein the pattern comprises a semi-permanent colorant and one or more compounds selected from the group of 1,3-butylene glycol, pentylene glycol, hexylene glycol or mixtures thereof.

In some embodiments according to the second aspect, the applicator may comprise a release liner, in particular a detachable release liner.

The content of the one or more compounds selected from the group of 1,3-butylene glycol, pentylene glycol, hexylene glycol or mixtures may be relatively low in the final applicator according to the second aspect. The applicator may undergo a heat treatment (which is discussed below), wherein a significant amount of the one or more compounds may evaporate. The one or more compounds may furthermore evaporate during storage. Nonetheless, it has been found that the inclusion of these one or more compounds in the temporary tattoo ink used to form the pattern (and which remain even in a low amount in the applicator), significantly reduces the patchiness after removal of the release liner, irrespective of a drying step following the printing. In some embodiments according to the second aspect, the pattern comprising the semi-permanent colorant may comprise between about 0.01 wt.-% to about 30% wt.-%, more specifically between about 0.05 wt.-% to about 25 wt.-%, and in particular between about 0.1 wt.-% to about 20 wt.-%, of the one or more compounds selected from the group of 1,3-butylene glycol, pentylene glycol, hexylene glycol or mixtures, relative to the total weight of the pattern. In some embodiments according to the second aspect, the pattern comprising the semi-permanent colorant may comprise between about 0.01 wt.-% to about 5% wt.-%, more specifically between about 0.05 wt.-% to about 3 wt.-%, and in particular between about 0.1 wt.-% to about 2 wt.-%, of the one or more compounds selected from the group of 1,3-butylene glycol, pentylene glycol, hexylene glycol or mixtures, relative to the total weight of the pattern.

In some embodiments according to the second aspect, the carrier liner has a first face and a second face, wherein the adhesive layer may be provided on at least about 50%, more specifically at least 70% and in particular at least 90% of at least the second face, relative to the total surface of the at least the second face. In particular, the adhesive layer may be provided on all of the at least second face of the carrier liner. The adhesive layer covering a high proportion of the surface of the second face of the carrier liner may improve the adhesion of the carrier to the skin when applied thereto.

In some embodiments according to the second aspect, the pattern may be provided on and/or in less than 95%, more specifically less than 90% and in particular less than 85% of the adhesive layer, relative to the total surface of the adhesive layer facing away from the carrier liner. In other words, the adhesive layer may have a first face and a second face, and the first face of the adhesive may be in contact with the second face of the carrier liner and the pattern may be provided on the second face of the adhesive. Hence, at least part of the adhesive layer is available to be brought into contact with skin. Further, the adhesive layer according to the second aspect may not include the pattern itself, instead the pattern is provided as a distinct entity. An adhesive layer including the pattern may allow less semi-permanent colorant to diffuse into the skin compared to a pattern provided on an adhesive layer. Yet, the pattern may have partially diffused into the adhesive layer.

The applicator may rely solely on residual solvents in the pattern and sweat to transfer the semi-permanent colorant from the pattern to the skin. In particular, the applicator may not require water to transfer the pattern, or semi-permanent colorant comprised within the pattern, to the skin. In some embodiments according to the second aspect, the carrier liner comprises, essentially consists or consists of a water-resistant polymer or a waterproof polymer, in particular a waterproof polymer. Accordingly, in some embodiments according to the second aspect, the carrier liner may be water-resistant or waterproof. The term "water-resistant carrier liner" within this disclosure refers to a carrier liner, that when in contact with a moist sponge cloth on a first face, does not show a detectable increase moisture on the second face after at least 10 minutes of contact. The term "waterproof carrier liner" within this disclosure refers to a carrier liner, that when in contact with a moist sponge cloth on a first face, does not show detectable increase in moisture on the second face after at least 1 hour of contact. The moist sponge cloth may be for example a sponge cloth comprising 75 cellulose and 25 % cotton, with a thickness in the dry state between about 2 mm to about 10 mm. The sponge cloth should be fully immersed in water for 5 minutes, and subsequently allowed to release excess water by being hung-up for 5 minutes, e.g. attached to a peg. For example, a "Vileda Original Schwammtuch" by the company Vileda GmbH, Weinheim, Germany, may be used for the test. The moisture may be detected using a moisture meter, e.g. a MM7 Pin-Type Moisture Meter purchasable from the company General Tool Inc, Irvine California. An increase of moisture may be considered a "detectable increase in moisture" if the moisture increases by at least 15%, more specifically at least 20%. The temperature of the moist sponge cloth needs to be at least ambient temperature, to prevent condensation of water on the second face of the carrier liner.

In some embodiments according to the second aspect, the adhesive layer may comprise a rubber adhesive, a silicone adhesive, a polyurethane adhesive, a hydrogel adhesive, a hydrocolloid adhesive, an acrylic adhesive and/or a dry adhesive. In particular, in some embodiments according to the second aspect, the adhesive layer comprises, essentially consists or consists of a rubber adhesive. The rubber adhesive may be for example a styrene-copolymer adhesive.

In some embodiments according to the second aspect, the adhesive layer may comprise a pressure-sensitive adhesive. A pressure-sensitive adhesive may be advantageous as it does not require activation, for example by water. The term "activation of an adhesive layer" refers to applying an external stimulus, except pressure, to the adhesive layer, for the adhesive layer to adhere to a surface, e.g. skin. The external stimulus may be one or more of heat, drying, shear, UV-light or the addition of other substances. In some embodiments according to the second aspect, the adhesive layer and/or pattern may be in direct contact with the release liner, more specifically the film and/or a coating provided or comprised in the film.

Further, the adhesive may be configured not to transfer to the skin. For example, decals may comprise an adhesive layer provided on a carrier liner and an additional water-absorbent layer. A water-soluble slip layer may be arranged between the water-absorbent layer, e.g. porous paper, and the carrier liner. The water-soluble slip layer may allow the adhesive layer and carrier to transfer from the water-absorbent layer to the skin upon application. However, the combination of the carrier liner with the adhesive layer may leave a sticky residue on the skin. In some embodiments according to the second aspect, the adhesive layer may be water-insoluble. The term "water-insoluble adhesive layer" within this disclosure may refer to an adhesive layer with a water-solubility of less than 0.25 g/dL, more specifically less than 0.1 g/dL and in particular less than 0.05 g/dL at 20 °C. In some embodiments according to the second aspect, the applicator may not comprise a water-soluble slip layer and/or a water-absorbent layer. Further, in some embodiments according to the second aspect, the carrier liner may be an outer layer of the applicator.

In some embodiments according to the second aspect, the adhesive layer may have a thickness between about 5 µm to about 200 µm, more specifically between about 20 µm to about 100 µm and in particular between about 30 µm to about 80 µm. The thickness may be measured from the carrier liner to the top of the adhesive layer, excluding the pattern. The thickness of the adhesive layer shall be measured at a part without pattern provided thereon.

As already explained above, the applicators of the present disclosure may be manufactured by inkjet printing. Applicators manufactured by inkjet printing, compared to for example applicators manufactured by screen printing, may be highly susceptible to patchiness, as they may be more delicate as they are applied in the form of tiny droplets, which dry to form spots.

In some embodiments according to the second aspect, the pattern may comprise a plurality of spots, wherein the plurality of spots may have an average maximum diameter between about 10 µm to about 500 µm, more specifically between about 50 µm to about 250 µm and in particular between about 75 µm to about 130 µm. The average maximum diameter of the spots may be determined using a microscope and averaged over 30 spots.

In some embodiments according to the second aspect, the release liner may be provided on and/or attached to the pattern. In some embodiments according to the second aspect, the release liner may be provided on and/or attached to the pattern and the adhesive layer, in particular on the opposite side of the adhesive layer whereto the carrier liner is attached. In some embodiments according to the second aspect, the release liner may comprise a non-adhesive material. In some embodiments according to the second aspect, the release liner may comprise a pull-tab to facilitate the detachment of the release liner. In some embodiments according to the second aspect, the release liner may comprise a polymer, in particular polyethylene terephthalate and/or a paper, in particular kraft paper. In some embodiments according to the second aspect, the release liner may comprise a film, in particular a polymer film. It has been found that a release liner comprising a film may result in less patchiness after removal of the release liner. It is believed that this is due to the lesser surface roughness of the film compared to paper. In some embodiments according to the second aspect, the average surface roughness of the film may be less than 3 µm, more specifically less than 1 µm and in particular less than 500 nm, measured according to ISO 21920-2:2021. In some embodiments according to the second aspect, the average surface roughness of the film may be between about 0.5 nm to about 500 nm, more specifically between about 1 nm to about 300 nm and in particular between about 3 nm to about 100 nm, measured according to ISO 21920-2:2021. In some embodiments according to the second aspect, the release liner may comprise a polymer or paper comprising a coating. For example, the release liner may comprise a silicone coating, more specifically a fluorosilicone and/or a non-halogenated silicone coating, in particular a polydimethylsiloxane coating. The silicone coating may be configured to reduce the adhesion between the release liner and the adhesive layer and/or pattern. It has been found that a silicone coating comprising, essentially consisting of a non-halogenated silicone, in particular polydimethylsiloxane, may result in a lower patchiness after removal compared to a fluorosilicone coating. Furthermore, fluorosilicones are an environmental concern. Furthermore, it has been found that the extractability of the silicone coating may influence the patchiness, more specifically that a higher extractability of the silicone coating may result in an increased patchiness, in particular after longer storage durations. Without wishing to be bound by theory, it is believed that silicone extractables may transfer from the silicone coating to the top of the pattern. The transferred silicone extractables may reduce the rate of genipin diffusion into the skin by forming a form of barrier. Accordingly, in some embodiments according to the second aspect the silicone coating may exhibit an extractability of less than 10 µg/cm² silicone, more specifically less than 1 µg/cm² silicone and in particular less than 100 ng/cm² silicone. In some embodiments according to the second aspect the silicone coating may exhibit an extractability of less than 5 wt.-% silicone, more specifically less than 3 wt.-% silicone and in particular less than 2 wt.-% silicone, relative to the total weight of the silicone coating. The extractability may be tested according to ISO 10993-18:2020. Alternatively or additionally, the extractability may be tested by submerging the release liner in a solvent selected from methylisobutylketone, xylene and toluene for 25 hours. Subsequently, the amount of silicone may be measured using atomic absorption spectrometry or inductively coupled plasma mass spectrometry. The total weight of the coating can be measured for example by X-ray fluorescence. Release liners may be sourced for example from the company Fox River Associates, Ponte Vedra, Florida, USA.

An exemplary exploded-view depiction of an applicator 100 according to the second aspect is shown in Fig.1. The applicator 100 of Fig. 1 comprises an adhesive layer 120 provided on a carrier liner 110. A pattern 130 is provided on the adhesive layer 120. The release liner 140 is provided on the pattern 130 and adhesive layer 120 (depicted with a distance for overview purposes).

In some embodiments according to the second aspect, the release liner may have a thickness between about 10 µm to about 400 µm, more specifically between about 30 µm to about 250 µm and in particular between about 40 µm to about 180 µm. As was outlined above, the applicators of the present disclosure may be manufactured by inkjet printing. For the inkjet printing process and subsequent drying process, the carrier liner may be provided with a support layer attached to the carrier liner, which increases the stiffness of the carrier liner. The increased stiffness may improve the processability of the carrier liner, e.g. by preventing wrinkling of the carrier liner. However, subsequently, the support layer needs to be removed from the applicator, as otherwise the applicator may not properly align to the user's skin due to the increased stiffness. However, during removal, e.g. by delamination, the applicator may wrinkle. A film of increased thickness, which is laminated onto the applicator prior to the delamination, may aid in preventing wrinkling of the applicator, e.g. by increasing the applicator's stiffness.

Further, the carrier liner with the adhesive layer may be provided in the form of large sheets which are printed at once and subsequently cut into the individual applicators. A film of greater thickness may also aid in allowing precise cutting without damage to the applicators. Further, the applicators may unintentionally curl after cutting due to absorption of water. The curling may be reduced using a thicker film. Finally, a release liner comprising or consisting of a film with increased thickness, may allow using a carrier liner of lesser thickness and/or higher flexibility, as it provides sufficient mechanical stability to the applicator. A thinner carrier liner may improve the alignment of the carrier liner to the skin.

In some embodiments according to the second aspect, the carrier liner may have a thickness between about 10 µm to about 300 µm, more specifically between about 30 µm to about 150 µm and in particular between about 40 µm to about 100 µm.

The pattern of the applicator according to the second aspect, may comprise all or some of the components of the temporary tattoo ink according to the second aspect. As mentioned above, the applicator according to the second aspect may undergo a heat treatment which may result in the evaporation of volatile and/or semi-volatile components of the temporary tattoo ink. Furthermore, volatile and/or semi-volatile components may evaporate during storage. In some embodiments according to the second aspect, the pattern may comprise the semi-permanent colorant. In some embodiments according to the second aspect, the pattern may comprise the one or more compounds selected from the group of 1,3-butylene glycol, pentylene glycol, hexylene glycol or mixtures thereof (albeit in trace amounts). In some embodiments according to the second aspect, the pattern may comprise the matrix component, the surface active agent, the one or more additional colorants, in particular the temporary colorant, the preservative, and/or one or more solvent (albeit again potentially in trace amounts). In some embodiments according to the second aspect, the pattern comprising the semi-permanent colorant may comprise between about 0.01 wt.-% to about 90% wt.-%, more specifically between about 0.05 wt.-% to about 85 wt.-%, and in particular between about 0.1 wt.-% to about 80 wt.-%, of the one or more solvents, relative to the total weight of the pattern. In some embodiments according to the second aspect, the pattern comprising the semi-permanent colorant may comprise between about 0.01 wt.-% to about 9% wt.-%, more specifically between about 0.05 wt.-% to about 5 wt.-%, and in particular between about 0.1 wt.-% to about 3 wt.-%, of the one or more solvents, relative to the total weight of the pattern.

In some embodiments according to the second aspect, the pattern comprising the semi-permanent colorant may comprise between about 1.5 wt.-% to about 30% wt.-%, more specifically between about 5 wt.-% to about 25 wt.-%, and in particular between about 10 wt.-% to about 20 wt.-%, of the matrix component, relative to the total weight of the pattern. In some embodiments according to the second aspect, the pattern comprising the semi-permanent colorant may comprise between about 5 wt.-% to about 30% wt.-%, more specifically between about 10 wt.-% to about 25 wt.-%, and in particular between about 15 wt.-% to about 20 wt.-%, of the matrix component, relative to the total weight of the pattern. In some embodiments according to the second aspect, the pattern comprising the semi-permanent colorant may comprise between about 30 wt.-% to about 90% wt.-%, more specifically between about 40 wt.-% to about 85 wt.-%, and in particular between about 50 wt.-% to about 75 wt.-%, of the semi-permanent colorant, relative to the total weight of the pattern.

In some embodiments according to the second aspect the applicator may have a shelf-life at ambient temperatures of at least 1 months, more specifically of at least 3 months and in particular of at least 6 months. It has been found, that the applicators according to the present disclosure, are particularly storage stable for a prolonged period of time.

### Process for obtaining the Applicator

In a third aspect, the present disclosure relates to an applicator obtained by applying a temporary tattoo ink on an adhesive layer provided on a carrier liner. The temporary tattoo ink comprises one or more semi-permanent colorants, and one or more compounds selected from the group of 1,3-butylene glycol, pentylene glycol, hexylene glycol or mixtures thereof.

In some embodiments according to the third aspect, the temporary tattoo ink may comprise one or more solvents, more specifically one or more polar solvents, and in particular the solvents may be selected from the group of water, ethanol or mixtures thereof.

In some embodiments according to the third aspect applying the temporary tattoo ink may be performed by printing, in particular inkjet printing. Inkjet printing is a precise, high-throughput method to produce the applicator. Further, inkjet printing allows to easily switch between patterns, allowing the provision of varying tattoo designs to the users.

In some embodiments according to the third aspect obtaining the applicator may additionally comprise a heat treatment, in particular following the application of the temporary tattoo ink on the adhesive layer. The heat treatment may dry the ink, reducing the risk of the ink being smudged subsequently. Further, the heat treatment may improve the adhesion between the pattern and the adhesive layer.

In some embodiments according to the third aspect the heat treatment may be performed at a temperature between about 45°C to about 85°C, more specifically between about 55°C to about 75°C for a duration of between about 20 min to about 120 min, in particular between about 30 min to about 60 min.

In some embodiments according to the third aspect obtaining the applicator may additionally comprise, in particular after the heat treatment, attaching a release liner to the adhesive layer, in particular by lamination. The release liner according to the third aspect may be in accordance with those defined above for the second aspect.

In some embodiments according to the third aspect the temporary tattoo ink may comprise between about 30 wt.-% to about 90 wt.-% of the one or more solvents, and/or between about 1 wt.-% to about 30 wt.-% of the one or more compounds, and/or between about 1 wt.-% to about 20 wt.-% of the one or more semi-permanent colorants.

In some embodiments according to the third aspect the one or more compounds may comprise pentylene glycol.

In some embodiments according to the third aspect, the temporary tattoo ink used to obtain the applicator may be a temporary tattoo ink according to the first aspect.

In some embodiments according to the third aspect the adhesive layer may comprise, essentially consist or consist of a rubber adhesive.

In a fourth aspect, the present disclosure relates to a method for manufacturing an applicator for applying a pattern comprising a semi-permanent colorant. The method comprises applying a temporary tattoo ink on an adhesive layer provided on a carrier liner, wherein the temporary tattoo ink comprises one or more semi-permanent colorants, and one or more compounds selected from the group of 1,3-butylene glycol, pentylene glycol, hexylene glycol or mixtures thereof.

In some embodiments according to the fourth aspect, the temporary tattoo ink may comprise one or more solvents, more specifically one or more polar solvents, and in particular the solvents may be selected from the group of water, ethanol or mixtures thereof.

In some embodiments according to the fourth aspect applying the temporary tattoo ink may be performed by printing, in particular inkjet printing.

In some embodiments according to the fourth aspect obtaining the applicator may additionally comprise a heat treatment, in particular following the application of the temporary tattoo ink. In some embodiments according to the fourth aspect the heat treatment may be performed at a temperature between about 45°C to about 85°C, more specifically between about 55°C to about 75°C for a duration of between about 20 min to about 120 min, in particular between about 30 min to about 60 min.

In some embodiments according to the fourth aspect obtaining the applicator may additionally comprise, in particular after the heat treatment, attaching a release liner to the adhesive layer, in particular by lamination. The release liner according to the fourth aspect may be in accordance with those defined above for the second aspect.

In some embodiments according to the fourth aspect the temporary tattoo ink may comprise between about 30 wt.-% to about 90 wt.-% of the one or more solvents, and/or between about 1 wt.-% to about 30 wt.-% of the one or more compounds, and/or between about 1 wt.-% to about 20 wt.-% of the one or more semi-permanent colorants.

In some embodiments according to the fourth aspect, the temporary tattoo ink used in the method, may be a temporary tattoo ink according to the first aspect.

In some embodiments according to the fourth aspect the one or more compounds may comprise pentylene glycol.

In some embodiments according to the fourth aspect the adhesive layer may comprise, essentially consist or consist of a rubber adhesive.

In a fifth aspect the present disclosure relates to the use of one or more compounds selected from the group of 1,3-butylene glycol, pentylene glycol, hexylene glycol and mixtures thereof in a temporary tattoo ink for preventing or reducing transfer of the printed temporary tattoo ink onto a release liner.

### Examples

Four different temporary tattoo inks were tested to assess the influence of different compounds on the patchiness of the resulting semi-permanent tattoo. The compositions are listed below in Table 1.

**Table 1: Tested temporary tattoo ink**

| Compositi ons | Water [wt.-%] | Ethanol [wt.-%] | Compound | Genipin [wt.-%] | Trehalose [wt.-%] | 1,2-hexane diol [wt.-%] | Euxyl PE 9010 [wt.-%] | Acid Blue 9 [wt.-%] |
|---|---|---|---|---|---|---|---|---|
| 1 | 29.91 | 42.87 | 14.96 wt.-% Pentylene Glycol | 6.98 | 1.99 | 1.99 | 1.00 | 0.30 |
| 2 | 29.91 | 42.87 | 14.96 wt.-% Transcutol | 6.98 | 1.99 | 1.99 | 1.00 | 0.30 |
| 3 | 23 | 61.2 | - | 11 | 1.5 | 2 | 1.00 | 0.30 |
| 4 | 29.91 | 42.87 | 14.96 wt.-% Propylene Glycol | 6.98 | 1.99 | 1.99 | 1.00 | 0.30 |

The viscosity of the inks was measured using a Brookfield Ametek DVE Viscometer, model no. DVEELVTJ0 with a UL adaptor for viscosities lower than 10 centipoise. The surface tension of the inks was measured using a Kruss Force Tensiometer using the du Noüy ring method according to ASTM D1331-20. The temporary tattoo ink 1 had a viscosity of 4.54 cP and a surface tension of 27.96 dyne/cm. The temporary tattoo ink 2 had a viscosity of 4.06 cP and a surface tension of 28.58 dynes/cm. The temporary tattoo ink 3 had a viscosity of 3.38 cP and a surface tension of 26.59 dynes/cm. The temporary tattoo ink 4 had a viscosity of 4.72 cP and a surface tension of 28.73 dynes/cm.

### Preparation of the Temporary Tattoo Ink

For the temporary tattoo inks of Table 1, the solids, (genipin and trehalose) were weighed out. The genipin was then dissolved in ethanol in a mechanical mixer at a speed of 800 rpm until fully dissolved (5 to 20 minutes) to form a first solution. The trehalose was dissolved in water in a mechanical mixer at a speed of 800 rpm until fully dissolved (5 to 20 minutes) to form a second solution. The first solution and second solution were then mixed with one another. Subsequently, the 1,2-hexanediol, Euxyl PE 9010 and acid blue 9 were added. Additionally, for the composition 1 pentylene glycol was added at this step, for the composition 2 transcutol and for the composition 4 propylene glycol was added at this step. The mixtures were then mixed in a mechanical mixer again for 20 minutes at 800 rpm. The resultant mixture was put onto a vacuum filter with 1.2 micron pore size and filtered into a sterilized polypropylene flask and transferred to sterilized polypropylene jars.

### Preparation of the Applicators

The temporary tattoo inks were printed by inkjet printing on a 2 mil rubber-based adhesive layer provided on a (2.9 mil) polyethylene carrier liner to form an applicator. The carrier liner was further supported by an 8 mil polypropylene backing. The temporary tattoo inks 1 to 3 were printed onto the adhesive layer in the form of a filled rectangle and in the form of a plurality of stripes adjacent to the rectangle. The temporary tattoo ink 4 was printed once in the form of a filled rectangle and a plurality of stripes adjacent to the rectangle, a tiger, a tree in a circle and paws in a heart and twice in the form of square.

Subsequently, the applicator was placed onto a plate and placed into a convection oven. The applicator underwent a heat treatment at 65 °C for 45 minutes. Subsequently, the sheets were left to cool at room temperature for 2 minutes.

Thereafter, a release liner (3.8 mil supercalendered kraft paper coated with fluorosilicone) was laminated onto the adhesive layer. Following lamination, the polypropylene backing layer was removed to acquire the final applicator samples.

### Patchiness testing of different temporary tattoo inks

The patchiness of the pattern following removal of the release liner was visually assessed. The release liner was removed after at least 15 days storage time for the temporary tattoo ink compositions 1 to 3. The release liner was removed after a storage time of 1 day and after 3 days for the temporary tattoo ink composition 4. The applicators were stored at 21°C ± 2°C and 30% relative humidity ± 10% relative humidity. The release liner was removed from the applicators by application of a continuous upwards pull. Subsequently, the patchiness of the pattern was analyzed by placing the carrier liner in front of a background light in the visible spectrum. Every composition was tested with three individual applicators. The following table 2 was used to assess the resulting pattern.

**Table 2**

| Patchiness Evaluation | |
|---|---|
| Little to no patchiness, high transparency in at least 80% pattern | 1 |
| Medium degree of patchiness | 2 |
| High degree of patchiness | 3 |
| Medium to no patchiness, however significant opaqueness in at least 30% of the overall pattern | 4 |

All samples manufactured from the temporary tattoo ink comprising 14.96 wt.-% pentylene glycol were graded as 1. All samples showed a low degree of patchiness, even after the relatively long storage time of at least 4 weeks. Some minor patchiness was observed in the plurality of stripes. Additionally, the printed pattern was transparent throughout, except for the patchy areas observed in the plurality of stripes.

Of the samples manufactured from the temporary tattoo ink comprising 14.96 wt.-% transcutol, two were graded as 3, while one was graded as 2. Two samples showed a plurality of opaque blots. Out of the two samples, one sample showed a transparent plurality of stripes, whereas the second sample showed a patchy plurality of stripes. The third sample showed a first transparent area taking up about half of the area of the filled rectangle, and a second patchy, mostly opaque, area taking up approximately the second half of the area of the filled rectangle. The plurality of stripes of the third sample showed significant patchiness.

All three samples manufactured from the temporary tattoo ink comprising no additional compound were graded as 4. All samples showed little to medium patchiness. However, except for some minor areas in the plurality of stripes, the printed pattern showed a significant opaqueness, indicating that the resulting semi-permanent tattoo would exhibit a lower color intensity.

For the fourth temporary tattoo ink, the release liner was removed from the filled rectangle and the plurality of stripes adjacent to the rectangle, the tiger and a first square after 1 day. The filled rectangle and in the form of a plurality of stripes adjacent to the rectangle and the tiger were graded as 1, the square was graded as 2. From tree in a circle, the paws in a heart and the second square, the release liner was removed after 3 days. The paws in a heart were graded as 2, the tree in a circle and the square was graded as 3. The test results show that propylene glycol is not as suitable to prevent patchiness, as is pentylene glycol, as the results are patchy already after three days.

### Correlation of Applicator's patchiness and Semi-permanent tattoo patchiness

To assess the correlation of the applicator's patchiness and the patchiness of the resulting Semi-permanent tattoo, a number of patchy and non-patchy applicators were tested. Photos were taken of the applicators prior to application to identify patchiness and regions of increased opaqueness. Subsequently, the applicators were applied to skin for 1 hour. Subsequently, the semi-permanent colorant was left to develop for 24 hours. After the 24 hours the skin was washed with water to remove any acid blue 9 dye which may still be present. Subsequently, the skin was visually assessed for patchiness. The results showed that the patchy applicators graded showed patchiness, whereas the non-patchy applicators showed low to no patchiness. Furthermore, by comparison of the skin with the photos of the applicators taken prior to the application to the skin, it was found, that the lighter areas of the semi-permanent tattoo on the skin, corresponded directly to the areas of increased opaqueness on the applicator.

### Embodiments

Although the present invention is defined in the attached claims, it should be understood that the present invention can also (alternatively) be defined in accordance with the following embodiments:
1. A temporary tattoo ink comprising:
   one or more semi-permanent colorants, and
   one or more compounds selected from the group of 1,3-butylene glycol, pentylene glycol, hexylene glycol or mixtures thereof.
2. The temporary tattoo ink according to embodiment 1, wherein the one or more compound comprises pentylene glycol.
3. The temporary tattoo ink according to any preceding embodiment, wherein the semi-permanent colorant comprises genipin or a genipin derivative, in particular purified genipin.
4. The temporary tattoo ink according to any preceding embodiment, wherein the temporary tattoo ink comprises one or more solvents, more specifically polar solvents, and in particular, wherein the solvents are selected from the group of water, ethanol or mixtures thereof.
5. The temporary tattoo ink according to any preceding embodiment, wherein the temporary tattoo ink comprises a surface active agent, more specifically wherein the surface active agent is a diol and in particular 1,2-hexanediol.
6. The temporary tattoo ink according to any preceding embodiment, wherein the temporary tattoo ink comprises a matrix component, more specifically wherein the matrix component is a polyol, even more specifically a disaccharide, and in particular trehalose.
7. The temporary tattoo ink according to any preceding embodiment, wherein the semi-permanent colorant is present in an amount between about 1 wt.-% to about 20 wt.-%, more specifically between about 3 wt.-% to about 15 wt.-% and in particular between about 5 wt.-% to about 10 wt.-%, relative to the total weight of the temporary tattoo ink.
8. The temporary tattoo ink according to any preceding embodiment, wherein the one or more compounds are present in an amount between about 1 wt.-% to about 30% wt.-%, more specifically between about 5 wt.-% to about 25 wt.-%, and in particular between about 10 wt.-% to about 20 wt.-%, relative to the total weight of the temporary tattoo ink.
9. The temporary tattoo ink according to any one of embodiments 4 to 8, wherein the one or more solvents are present in an amount between about 30 wt.-% to about 90% wt.-%, more specifically between about 50 wt.-% to about 85 wt.-%, and in particular between about 65 wt.-% to about 80 wt.-%, relative to the total weight of the temporary tattoo ink.
10. The temporary tattoo ink according to any one of embodiments 4 to 9, wherein the solvent comprises water and ethanol, more specifically wherein the ratio between water and ethanol is between about 1:1 to about 1:2, more specifically between about 1:1.3 to about 1:1.8.
11. The temporary tattoo ink according to any one of embodiments 6 to 10, wherein the matrix component is present in an amount between about 0.1 wt.-% to about 6 % wt.-%, more specifically between about 0.5 wt.-% to about 4 wt.-%, and in particular between about 1 wt.-% to about 3 wt.-%, relative to the total weight of the temporary tattoo ink.
12. An applicator for applying a pattern comprising a semi-permanent colorant, wherein the applicator comprises:
   a carrier liner and an adhesive layer,
   and a pattern provided on and/or in the adhesive layer,
   wherein the pattern comprises a semi-permanent colorant and one or more compounds selected from the group of 1,3-butylene glycol, pentylene glycol, hexylene glycol or mixtures thereof.
13. The applicator according to embodiment 12, wherein the carrier liner has a first face and a second face, wherein the adhesive layer is provided on at least about 50%, more specifically at least 70% and in particular at least 90% of the second face, relative to the total surface of the second face.
14. The applicator according to embodiment 12 or 13, wherein the pattern is provided on and/or in less than 95%, more specifically less than 90% and in particular less than 85% of the adhesive layer, relative to the total surface of the adhesive layer facing away from the carrier liner.
15. The applicator according to any one of embodiments 12 to 14, wherein the adhesive layer comprises, essentially consists or consists of a rubber adhesive.
16. The applicator according to any one of embodiments 12 to 15, wherein the applicator comprises a release liner.
17. The applicator according to any one of embodiments 12 to 16, wherein the pattern comprises a plurality of spots, wherein the plurality of spots has an average maximum diameter between about 10 µm to about 500 µm, more specifically between about 50 µm to about 250 µm and in particular between about 75 µm to about 130 µm.
18. The applicator according to any one of embodiments 12 to 17, wherein the carrier liner has a thickness between about 10 µm to about 300 µm, more specifically between about 30 µm to about 150 µm and in particular between about 40 µm to about 100 µm.
19. The applicator according to any one of embodiments 12 to 18, wherein the carrier liner comprises, essentially consists or consists of a water-resistant polymer or a waterproof polymer, in particular a waterproof polymer.
20. The applicator according to any one of embodiments 12 to 19, wherein the carrier liner is water-resistant or waterproof.
21. The applicator according to any one of embodiments 12 to 20, wherein the adhesive layer comprises a pressure-sensitive adhesive.
22. The applicator according to any one of embodiments 12 to 21, wherein the adhesive layer has a thickness between about 5 µm to about 200 µm, more specifically between about 20 µm to about 100 µm and in particular between about 30 µm to about 80 µm.
23. The applicator according to any one of embodiments 12 to 22, wherein the pattern comprising the semi-permanent colorant comprises between about 0.01 wt.-% to about 30% wt.-%, more specifically between about 0.05 wt.-% to about 25 wt.-%, and in particular between about 0.1 wt.-% to about 20 wt.-%, of the one or more compounds, relative to the total weight of the pattern.
24. The applicator according any one of embodiments 12 to 23, wherein the pattern comprises a matrix component, in particular wherein the matrix component comprises a polyol.
25. The applicator according to embodiment 24, wherein the polyol has a molar mass of at least 100 g/mol, more specifically at least 200 g/mol, and in particular at least 300 g/mol. The matrix component may be solid at 0°C, more preferably a solid at 20°C, still more specifically a solid at 30°C, in particular solid at 45°C.
26. The applicator according to any one of embodiments 12 to 25, wherein the pattern comprising the semi-permanent colorant comprises between about 0.01 wt.-% to about 90% wt.-%, more specifically between about 0.05 wt.-% to about 85 wt.-%, and in particular between about 0.1 wt.-% to about 80 wt.-%, of the one or more solvents, relative to the total weight of the pattern.
27. An applicator obtained by:
   - applying a temporary tattoo ink on an adhesive layer provided on a carrier liner, wherein the temporary tattoo ink comprises:
      one or more semi-permanent colorants,
      one or more compounds selected from the group of 1,3-butylene glycol, pentylene glycol, hexylene glycol or mixtures thereof.
28. The applicator according to embodiment 27, wherein applying the temporary tattoo ink is performed by printing, in particular inkjet printing.
29. The applicator according to embodiment 27 or 28, wherein obtaining the applicator additionally comprises a heat treatment, in particular following the application of the temporary tattoo ink to the adhesive layer.
30. The applicator according to embodiment 29, wherein the heat treatment is performed at a temperature between about 45°C to about 85°C, in particular between about 55°C to about 75°C for a duration of between about 20 min to about 120 min, in particular between about 30 min to about 60 min.
31. The applicator according to any one of embodiments 27 to 30, wherein obtaining the applicator additionally comprises, in particular after the heat treatment, attaching a release liner to the adhesive layer, in particular by lamination.
32. The applicator according to any one of embodiments 27 to 31, wherein the temporary tattoo ink comprises:
   between about 30 wt.-% to about 90 wt.-% of one or more solvents, and/or
   between about 1 wt.-% to about 30 wt.-% of the one or more compounds, and/or
   between about 1 wt.-% to about 20 wt.-% of the one or more semi-permanent colorants.
33. The applicator according to any one of embodiments 27 to 32, wherein the one or more compounds comprise pentylene glycol.
34. The applicator according to any one of embodiments 27 to 33, wherein the adhesive layer comprises, essentially consists or consists of a rubber adhesive.
35. A method for manufacturing an applicator for applying a pattern comprising a semi-permanent colorant, wherein the method comprises:
   - applying a temporary tattoo ink on an adhesive layer provided on a carrier liner, wherein the temporary tattoo ink comprises:
      one or more semi-permanent colorants,
      one or more compounds selected from the group of 1,3-butylene glycol, pentylene glycol, hexylene glycol or mixtures thereof.
36. Use of one or more compounds selected from the group of 1,3-butylene glycol, pentylene glycol, hexylene glycol and mixtures thereof in a temporary tattoo ink for preventing or reducing transfer of the printed temporary tattoo ink onto a release liner.

## Claims

1. A temporary tattoo ink comprising:
one or more semi-permanent colorants, and
one or more compounds selected from the group of 1,3-butylene glycol, pentylene glycol, hexylene glycol or mixtures thereof.

2. The temporary tattoo ink according to claim 1, wherein the one or more compounds comprises pentylene glycol.

3. The temporary tattoo ink according to any preceding claim, wherein the semi-permanent colorant comprises genipin or a genipin derivative, in particular purified genipin.

4. The temporary tattoo ink according to any preceding claim, wherein the temporary tattoo ink comprises a matrix component, more specifically wherein the matrix component is a polyol, even more specifically a disaccharide, and in particular trehalose.

5. The temporary tattoo ink according to any preceding claim, wherein the one or more compounds are present in an amount between about 1 wt.-% to about 30% wt.-%, more specifically between about 5 wt.-% to about 25 wt.-%, and in particular between about 10 wt.-% to about 20 wt.-%, relative to the total weight of the temporary tattoo ink.

6. An applicator for applying a pattern comprising a semi-permanent colorant, wherein the applicator comprises:
a carrier liner and an adhesive layer,
and a pattern provided on and/or in the adhesive layer,
wherein the pattern comprises a semi-permanent colorant and one or more compounds selected from the group of 1,3-butylene glycol, pentylene glycol, hexylene glycol or mixtures thereof.

7. The applicator according to claim 6, wherein the carrier liner has a first face and a second face, wherein the adhesive layer is provided on at least about 50%, more specifically at least 70% and in particular at least 90% of the second face, relative to the total surface of the second face.

8. The applicator according to claim 6 or 7, wherein the pattern is provided on and/or in less than 95%, more specifically less than 90% and in particular less than 85% of the adhesive layer, relative to the total surface of the adhesive layer facing away from the carrier liner.

9. The applicator according to any one of claims 6 to 8, wherein the applicator comprises a release liner.

10. An applicator obtained by:
- applying a temporary tattoo ink on an adhesive layer provided on a carrier liner, wherein the temporary tattoo ink comprises:
one or more semi-permanent colorants,
one or more compounds selected from the group of 1,3-butylene glycol, pentylene glycol, hexylene glycol or mixtures thereof.

11. The applicator according to claim 10, wherein applying the temporary tattoo ink is performed by printing, in particular inkjet printing.

12. The applicator according to claim 10 or 11, wherein the pattern comprises a plurality of spots, wherein the plurality of spots has an average maximum diameter between about 10 µm to about 500 µm, more specifically between about 50 µm to about 250 µm and in particular between about 75 µm to about 130 µm.

13. The applicator according to any one of claims 10 to 12, wherein the temporary tattoo ink comprises:
between about 30 wt.-% to about 90 wt.-% of one or more volatile solvents, and/or
between about 1 wt.-% to about 30 wt.-% of the one or more compounds, and/or
between about 1 wt.-% to about 20 wt.-% of the one or more semi-permanent colorants.

14. A method for manufacturing an applicator for applying a pattern comprising a semi-permanent colorant, wherein the method comprises:
- applying a temporary tattoo ink on an adhesive layer provided on a carrier liner, wherein the temporary tattoo ink comprises:
one or more semi-permanent colorants,
one or more compounds selected from the group of 1,3-butylene glycol, pentylene glycol, hexylene glycol or mixtures thereof.

15. Use of one or more compounds selected from the group of 1,3-butylene glycol, pentylene glycol, hexylene glycol and mixtures thereof in a temporary tattoo ink for preventing or reducing transfer of the printed temporary tattoo ink onto a release liner.
